# EUROPÄISCHE PATENTANMELDUNG

(11) **EP 1 803 702 A1**
(43) Veröffentlichungstag der Anmeldung: **04.07.2007**
(21) Anmeldenummer: 05113084.7
(22) Anmeldetag: 30.12.2005
(51) Int. Cl.: C07C 45/00, C07C 49/167, C07C 51/62

(54) **Drucklose Anlagerung von Acylfluoriden an Perfluoralkene**

(71) Anmelder: Solvay Fluor GmbH, 30173 Hannover (DE)
(72) Erfinder: Wiesenhöfer, Wolfgang Dr., 40885 Ratingen (DE); Hausmann, Eckhard, 30519, Hannover (DE); Kalbreyer, Wolfgang, 31595, Steyerberg (DE); Claassen, Uta, 31249, Hohenhameln (DE); Eichholz, Kerstin, 30855, Langenhagen (DE); Eicher, Johannes Dr., 31319 Sehnde (DE)
(74) Vertreter: Jacques, Philippe

(57) **Zusammenfassung**

Carbonylfluorid und Carbonsäurefluoride können unter drucklosen Bedingungen an Perfluoralkene angelagert werden, beispielsweise an Hexafluorpropen. Auf einfache Weise lassen sich so Perfluoralkylcarbonsäurefluoride bzw. Ketone mit einer Perfluoralkylgruppe herstellen.

## Beschreibung

Die Erfindung bezieht sich auf ein Verfahren zur Herstellung von Verbindungen mit einer Perfluoralkylcarbonylgruppe durch Anlagerung von Carbonylfluorid oder Carbonsäurefluoriden an Perfluoralkene.

Fluorierte Carbonylverbindungen sind Zwischenprodukte in der chemischen Synthese. Perfluoralkylketone und Perfluoralkylalkylketone sind beispielsweise als Feuerlöschmittel brauchbar, siehe WO 01/05468. Fluorierte Carbonsäurefluoride sind ebenfalls Zwischenprodukte in der chemischen Synthese, beispielsweise bei der Herstellung von Ketonen, Carbonsäuren und Carbonsäurederivaten wie Ester oder Amide.

Die Herstellung von fluorierten Ketonen und Carbonsäurefluoriden durch Anlagerung von Carbonylfluorid (COF₂) oder Carbonsäurefluoriden an perfluorierte Alkene ist aus den Publikationen von F.S. Fawcett, C.W. Tullock und D.D. Coffman in J. Am. Chem. Soc. 84 (1962), Seiten 4275 bis 4285, und R.D. Smith, F.S. Fawcett und D.D. Coffman in J. Am. Chem. Soc. 84 (1962), Seiten 4285 bis 4288 bekannt. Dabei werden Carbonylfluorid oder fluorierte Carbonsäurefluoride wie Trifluoracetylfluorid, n-Perfluorpropylcarbonsäurefluorid, i-Perfluorpropylcarbonsäurefluorid oder ω-H-Oktafluorbutylcarbonsäurefluorid an Tetrafluorethylen, Hexafluorpropen oder Oktafluorbuten addiert. Cesiumfluorid, Kaliumfluorid oder Kaliumbifluorid sind als Katalysatoren genannt. Die Reaktion wird unter autogenem Druck durchgeführt. Auch das US-Patent 3185734 beschreibt eine solche, unter autogenem Druck durchgeführte Reaktion. Dafür sind spezielle Druckgefäße nötig.

Aufgabe der vorliegenden Erfindung ist es, ein vereinfachtes Verfahren zur Herstellung von Carbonsäurefluoriden und fluorierten Ketonen durch Addition von Carbonylfluorid oder Carbonsäurefluoriden an Perfluoralkene anzugeben. Diese Aufgabe wird durch das Verfahren der vorliegenden Erfindung gelöst. Das erfindungsgemäße Verfahren sieht vor, dass man perfluorierte Carbonsäurefluoride und fluorierte Ketone mit einer Perfluoralkylgruppe durch Anlagerung von Carbonylfluorid oder Carbonsäurefluoriden an perfluorierte Alkene herstellt, wobei die Stufe der Anlagerung drucklos durchgeführt wird.

Die Addition von Carbonsäurefluoriden an perfluorierte Alkene ergibt Ketone. Bei der Umsetzung von Carbonylfluorid ist bei geeigneter Wahl des Molverhältnisses von Alken zu Carbonylfluorid lässt sich das Carbonylfluorid an zwei Moleküle des Alkens addieren, und es werden Ketone gebildet. Die ideale Stöchiometrie gemäß Reaktionsgleichung für diese Reaktion ist 2:1. Aus Carbonylfluorid und Alken mit ensprechend angepasstem Molverhältnis entstehen Carbonsäurefluoride. Die ideale Stöchiometrie gemäß Reaktionsgleichung für diese Umsetzung ist 1:1. Die Herstellung von Ketonen aus Alken und Carbonsäurefluorid ist im Rahmen der vorliegenden Erfindung bevorzugt.

Perfluorierte Alkene entsprechen im Rahmen der vorliegenden Erfindung bevorzugt der Formel (1), R¹-CF=CFR². R¹ und R² sind gleich oder verschieden und bedeuten perfluoriertes C1-C4-Alkyl oder Fluor. Bevorzugt bedeutet R¹ Fluor, Perfluormethyl oder Perfluorethyl, und R² bedeutet bevorzugt Fluor, Perfluormethyl oder Perfluorethyl. Ganz besonders bevorzugt ist das perfluorierte Alken Hexafluorpropen.

Bevorzugte Carbonsäurefluoride sind solche der Formel R³-C(O)F (II), wobei R³ für einen C1-C5-Alkylrest steht, der gewünschtenfalls durch mindestens 1 Halogenatom substituiert ist. Bevorzugt steht R³ für C1-C3-Alkyl, das durch mindestens 1 Fluoratom substituiert ist. Ganz besonders bevorzugt steht R³ für CF_{3,} CHF₂ oder CF₂Cl.

Das erfindungsgemäße Verfahren ist hervorragend zur Herstellung von Trifluormethylperfluorisopropylketon aus Trifluoracetylfluorid und Hexafluorpropen geeignet.

Die Umsetzung wird in einem polaren aprotischen Lösungsmittel durchgeführt. Vorteilhaft ist eine hohe Dielektrizitätskonstante. Solche Lösemittel besitzen auch die Fähigkeit, anorganische Salze zu lösen. Bevorzugte Lösungsmittel besitzen einen Siedepunkt, der mindestens 50 °C, bevorzugt mindestens 70 °C beträgt. Sehr gut geeignet sind Dialkylamide der niederen Carbon- oder Fettsäuren wie Dimethylformamid, Dialkylether, Alkylen- oder Polyalkylenglycole, flüssige Nitrile wie Acetonitril, Propionitril, Butyronitril, Valeronitril, Benzonitril, Sulfolan, sowie ionische Flüssigkeiten.

Das erfindungsgemäße Verfahren wird bevorzugt bei einer Temperatur von mindestens 50 °C, besonders bevorzugt von mindestens 70 °C durchgeführt. Die obere Grenze ist variabel und vom Siedepunkt des Lösungsmittels bestimmt. Eine obere Grenze von 170 °C ist bevorzugt.

Es ist von Vorteil, wenn die Reaktanten möglichst fein verteilt in das Lösungsmittel bzw. die Reaktionsmischung eingeleitet werden. Dies kann z.B. bewirkt werden, indem man die Reaktanten, besonders wenn sie gasförmig sind, durch eine Fritte einleitet.

Der Begriff "drucklos" bedeutet, dass außer dem Förderdruck der zuzuführenden Verbindungen kein Druck auf die Reaktionsmischung einwirkt, auch kein autogener Druck. Der Druck liegt somit im Bereich von Atmosphärendruck (etwa 1 bar abs.) bis maximal 1,5 bar (abs.).

Als Katalysator kann ein Fluorid verwendet werden, bevorzugt ein quaternäres Ammoniumfluorid oder ein Alkalimetallfluorid wie KF oder CsF. Vorteil dieses Reaktionssystems ist u. a. eine geringe Korrosivität, d. h. dass trotz der Anwesenheit hoher Fluoridkonzentration kann in Glas gearbeitet werden kann ohne dass sichtbare Glasätze eintritt.
Das Verfahren wird bevorzugt kontinuierlich durchgeführt.

Vorteil des erfindungsgemäßen Verfahrens ist, dass es kontinuierlich durchgeführt werden kann und keine Druckapparate benötigt werden.. Dadurch können große Mengen in Standardapparaturen hergestellt werden, wodurch hohe Investitionskosten vermieden werden. Durch die kontinuierliche Verfahrensweise ist die Raumzeitausbeute wesentlich erhöht (im Vergleich zu den Beispielen aus US3185734 um den Faktor 7). Ausbeute und Selektivität sind sehr gut.

Die folgenden Beispiele sollen die Erfindung weiter erläutern, ohne sie in ihrem Umfang einzuschränken.

### Beispiele:

### Beispiel 1: Herstellung von Trifluormethylperfluorisopropylketon

In einem zylindrischen 200 ml-Glasgefäß mit Magnetrührstäbchen wurden 2,8 g KF (0,05 mol) in 108,2 g DMF (Dimethylformamid) angeschlämmt. Das Gemisch wurde im Wasserbad auf ca. 80 °C erwärmt und durch einen 1/8" FEP-Schlauch (FEP steht für "Fluorinated Ethylene Propylene tubing") 18,3 g Trifluoracetylfluorid (TFAF; 0,16 mol) und 18,7 g Hexafluorpropen (0,12 mol) mit einem Feed von 5,23 g TFAF / h und 5,34 g HFP / h eingeleitet. Die Destillationsbrücke wurde bei -10 °C betrieben. Das Kondensat wurde in einer eisgekühlten Vorlage aufgefangen. Das Abgas wurde durch einen Edelstahlzylinder im Trockeneiskühlbad geleitet.

Kondensat in der Vorlage: 18,4 g (GC 81,9 Flächen-%) ≈ 0,06 mol ≈ 47,2 % d. Th.
Kondensat in der Kühlfalle: 2,8 g (GC 45,5 Flächen-%).%) ≈ 0,005 mol

Die Gesamtausbeute an Trifluormethylperfluorisopropylketon betrug somit 54 %.

### Beispiel 2: Herstellung von Trifluormethylperfluorisopropylketon

Der Versuch wurde unter Verwendung des in Beispiel 1 benutzten Gemisches von KF und DMF wiederholt. Die Gase wurden jedoch durch eine Fritte geleitet. 43,2 g TFAF (0,37 mol) wurden so mit 19,2 g HFP (0,13 mol) bei einem Feed von 24,7 g TFAF / h und 11,0 g HFP / h umgesetzt.
Kondensat in der Vorlage: 23,3 g (GC 86,9 Flächen-%) ≈ 0,08 mol ≈ 58,5 % d. Th.
Kondensat in der Kühlfalle: 13,1 g (GC 67,1 Flächen-%) ≈ 0,03 mol ≈ 25,4 % d.
Th.

Hier betrug die Ausbeute somit insgesamt 84% der Theorie.

Das Beispiel belegt, dass trotz der viel einfacheren und zeitsparenden Verfahrensdurchführung eine Ausbeute zu erzielen ist, die derjenigen des Standes der Technik, z.B. gemäß Beispiel II der US-A 3185734, mindestens gleichkommt. Die Raumzeitausbeute ist für das neue Verfahren deutlich erhöht (ca. um dem Faktor 7).

### Beispiel 3: Herstellung von Trifluormethylperfluorisopropylketon

In einer Technikumsapparatur (100 l emaillierter beheizbarer Stahlbehälter, mit aufgesetzter Kolonne, Rührer und Pumpenumlauf) wurden 0,58 kg Kaliumfluorid (KF) und 36,5 kg Dimethylformamid (DMF) eingefüllt und unter Rühren auf 80 °C temperiert. Sodann wurde der Pumpenumlauf eingeschaltet und die Edukte Hexafluorpropen (HFP) und Trifluoracetylfluorid (TFAF) in jenen eingeleitet. Das Produkt wurde aus dem Reaktor destilliert und in gekühlten Vorlagen aufgefangen. Es wurden mit derselben DMF-KF-Charge drei Experimente durchgeführt, die in der folgenden Tabelle beschrieben werden:

| Experiment [Nr.] | Dauer [h] | HFP | | | TFAF | | | Auswaage [kg] | Gehalt [%] |
|---|---|---|---|---|---|---|---|---|---|
| | | [kg] | [mol] | [kg/h] | [kg] | [mol] | [kg/h] | | |
| 1 | 1,67 | 2,0 | 13,3 | 1,2 | 2,6 | 22,6 | 1,6 | -^{a} | 93,3 |
| 2 | 6 | 7,0 | 47,0 | 1,2 | 6,0 | 51,9 | 1,0 | 9,6 | 93,4 |
| 3 | 3,5 | 4,7 | 31,0 | 1,3 | 3,6 | 30,8 | 1,0 | 9,0 | 91,8 |
| Summe | 11,17 | 13,7 | 91,3 | - | 12,2 | 105,3 | - | 18,6 | - |

| | | | | | | | | | |
|---|---|---|---|---|---|---|---|---|---|
| ^{a} nur Probe gezogen, Auswaage dieses Versuches ist im Experiment 2 enthalten | | | | | | | | | |

Die Gesamtrohausbeute beträgt 71 %

Das Beispiel belegt, dass größere Mengen Keton in Standardapparaturen hergestellt werden können und somit große Investitionskosten für Spezialreaktoren vermieden werden können.

## Patentansprüche

1. Verfahren zur Herstellung von perfluorierten Carbonsäurefluoriden und fluorierten Ketonen mit einer Perfluoralkylgruppe durch Anlagerung von Carbonylfluorid oder Carbonsäurefluoriden an perfluorierte Alkene, wobei die Stufe der Anlagerung drucklos durchgeführt wird.

2. Verfahren nach Anspruch 1, **dadurch gekennzeichnet, dass** man Carbonsäurefluoride mit einem perfluorierten Alken umsetzt und ein fluoriertes Keton mit einer Perfluoralkylgruppe herstellt.

3. Verfahren nach Anspruch 1, **dadurch gekennzeichnet, dass** man dass man Carbonylfluorid mit einem perfluorierten Alken umsetzt und in Abhängigkeit des Molverhältnisses Carbonsäurefluoride oder Ketone herstellt.

4. Verfahren nach Anspruch 1, **dadurch gekennzeichnet, dass** das perfluorierte Alken eine Verbindung der der Formel (I), R¹-CF=CFR² ist, wobei R¹ und R² gleich oder verschieden sind und perfluoriertes C1-C4-Alkyl oder Fluor bedeuten.

5. Verfahren nach Anspruch 4, **dadurch gekennzeichnet, dass** R¹ Perfluormethyl oder Perfluorethyl bedeutet, und R² Fluor, Perfluormethyl oder Perfluorethyl bedeutet.

6. Verfahren nach Anspruch 5, **dadurch gekennzeichnet, dass** das perfluorierte Alken Hexafluorpropen ist

7. Verfahren nach Anspruch 1, **dadurch gekennzeichnet, dass** Carbonsäurefluoride der Formel R³-C(O)F (II) eingesetzt werden, wobei R³ für einen C1-C5-Alkylrest steht, der gewünschtenfalls durch mindestens 1 Halogenatom substituiert ist.

8. Verfahren nach Anspruch 7, **dadurch gekennzeichnet, dass** R³ für C1-C3-Alkyl steht, das durch mindestens 1 Fluoratom substituiert ist.

9. Verfahren nach Anspruch 8, **dadurch gekennzeichnet, dass** R³ für CF₃, CHF₂ oder CF₂Cl steht.

10. Verfahren nach Anspruch 1, **dadurch gekennzeichnet, dass** die Umsetzung in einem Lösungsmittel mit einem Siedepunkt von mindestens 50 °C, vorzugsweise von mindestens 70 °C bei 1 bar (abs.) durchgeführt wird, vorzugsweise in Dimethylformamid.

11. Verfahren nach Anspruch 1, **dadurch gekennzeichnet, dass** die Umsetzung bei einer Temperatur von mindestens 50 °C, vorzugsweise von mindestens 70 °C, und maximal 170 °C durchgeführt wird.

12. Verfahren nach Anspruch 1, **dadurch gekennzeichnet, dass** die Umsetzung kontinuierlich durchgeführt wird.
